# EUROPEAN PATENT APPLICATION

(11) **EP 3 949 902 A1**
(43) Date of publication of application: **09.02.2022**
(21) Application number: 19923680.3
(22) Date of filing: 04.04.2019
(51) Int. Cl.: A61F 2/16, G02C 7/04

(54) **SUPPLEMENTARY INTRAOCULAR LENS**

(71) Applicant: Palomino Munoz, Antonio, 28009 Madrid (ES); Fernández Ruiz, Alfredo Julián, 13005 Ciudad Real (ES); Adan Ruiz, Antonio, 13002 Ciudad Real (ES)
(72) Inventor: Palomino Munoz, Antonio, 28009 Madrid (ES); Fernández Ruiz, Alfredo Julián, 13005 Ciudad Real (ES); Adan Ruiz, Antonio, 13002 Ciudad Real (ES)
(74) Representative: Álvarez López, Sonia
(86) International application number: PCT/ES2019/070229
(87) International publication number: WO 2020/201588

(57) **Abstract**

Intraocular lens (1), comprising:
- a lenticular body (2) of a diameter similar to the inner diameter of the capsular bag (3) of the lens or the sulcus (4),
- whose thickness is between 0.05-0.5 millimetres, and
- an extender element of said lenticular body (2).

The body (2) may have a pupillary orifice and be coloured as a natural iris, a pinhole, or be completely opaque.

## Description

### OBJECT OF THE INVENTION

The present invention relates to an intraocular lens (IOL) intended to be supplemented on another intraocular lens.

### BACKGROUND OF THE INVENTION

Currently, intraocular lenses (lOLs) used to correct different vision deficiencies (cataracts, refractive deficiencies, etc.) comprise a graduated lenticular optic or body provided with expansive haptic handles, which are merely centring devices that ensure a centred position of the IOL inside the capsular sac of the lens, which is usually its placement destination, although sometimes, when said sac is broken, it can be placed in the sulcus, which is the groove behind the pupil.

These intraocular lenses have a diameter less than that of the capsular bag, of about 6 or 8 millimetres maximum, and a thickness of about 0.7-0.75 millimetres to ensure stability or rigidity that prevents the lens from bending or deforming due to the supporting force of the expanding handles against the sulcus or capsular bag.

This size, thickness and form of fastening, however, sometimes lead to certain limitations, such as not covering the entire diameter of the sulcus, and therefore they-cannot be used to simulate an iris when wishing to correct total or partial loss of said iris.

The total or partial loss of the iris causes different problems to the patient, starting with an aesthetic problem, a light protection problem (it is very uncomfortable, almost unbearable, to lack this protection, and especially in situations with a lot of sunlight) as well as a refractive problem.

This drawback is solved by the use of the lens of the invention.

### DESCRIPTION OF THE INVENTION

The intraocular supplementary lens of the invention has a configuration that allows covering the entire diameter of the sulcus and sac and solving simulation problems of a complete iris.

According to the invention, the supplementary lens comprises:
- a lenticular body with a diameter similar to the inner diameter of the sulcus (approximately 12 mm)
- whose thickness is between 0.05-0.5 millimetres, and
- including also an extensor element of said lenticular body

In this way, thanks to its amplitude, its reduced thickness, and the extender element, the lens of the invention can function as a supplementary lens which can be placed together with another intraocular lens to solve problems such as the simulation of an iris to correct the absence or loss of the natural iris.

In addition, in the search for this supplementary lens capable of covering the entire diameter of the sulcus to correct these deficiencies in the iris (total or partial loss of iris), it has been found that it can be inserted as a supplementary lens both in the sulcus (for pseudophakic patients, that is, already provided with an anterior IOL), and in the capsule of the lens together with an intraocular lens (for phakic patients) thanks to its reduced thickness and the stability given by the extensor element, serving to correct different deficiencies that are detailed below

Thus, within the invention, it has been foreseen that the lenticular body may also have a diameter similar to that of the capsular sac of the lens (about 10 mm). In any version (for a sulcus with a diameter of about 9-11 millimetres, or for a capsular sac with a diameter of about 11-13 millimetres) these are diameters greater than that of current intraocular lenses.

To implant it together with another intraocular lens in a cataract operation of a phakic patient (who has their original lens, which will be removed in this intervention), conventional cataract surgery would be performed with the injection of a typical IOL (intraocular lens) in the sac, plus the introduction of our supplementary lens with intraocular contact, extension and placement of the same with viscoelastic, securing its position with the extensor element. If the patient is pseudophakic (that is, having a preimplanted IOL) and we cannot place it in the capsular sac due to fibrosis, we will place our supplementary sulcus lens in the same way with the same procedure. That is, the invention features a supplementary lens, which is placed on top of the normal intraocular lens in the same cataract surgery intervention and inside the capsular bag, or if the patient had undergone a previous cataracts operation, we would put it in the sulcus.

Thus, as the supplementary lens of the invention is so thin, it is possible to:
1) fit it easily inside the capsular sac above the classic intraocular lens, which can be done because stability is ensured as it occupies the entire capsular sac and by the extensor element, unlike in current lenses where in reality their haptic handles are expansive/centring
2) insert it into the eye simply with a clamp without the need for special injectors
3) extract it in case of intolerance is much more easily because we would not manipulate the intraocular lens that the patient has in place.

### BRIEF DESCRIPTION OF THE DRAWINGS

Figure 1 shows the supplementary lens of the invention, showing the lenticular body and the extender element placed in the lenticular body and fastened by the haptic handles secured with a capsular ring.
Figure 2 shows a first variant of the lenticular body of the supplementary lens of the invention for correction or supplementation of an iris.
Figure 3 shows a second variant of the lenticular body of the supplementary lens of the invention with pinhole for astigmatism correction.
Figure 4 shows a third variant of the lenticular body of the supplementary lens of the invention for presbyopia or ametropia correction.
Figure 5 shows a fourth variant of the lenticular body of the supplementary lens of the invention for incorrigible diplopias.
Figure 6 shows the supplementary lens of the invention, in any of the above variants, placed in the sulcus.
Figure 7 shows the supplementary lens of the invention, in any of the above variants, placed in the capsular bag.

### DESCRIPTION OF A PRACTICAL EMBODIMENT OF THE INVENTION

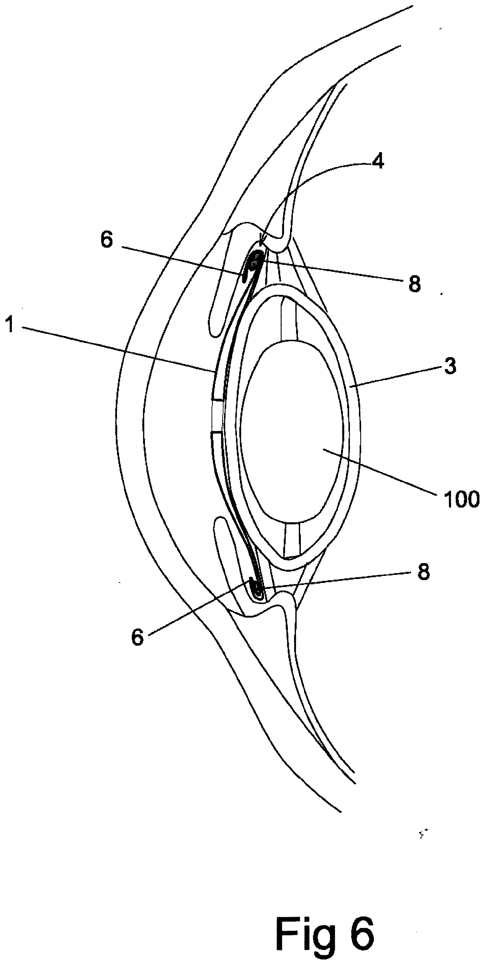

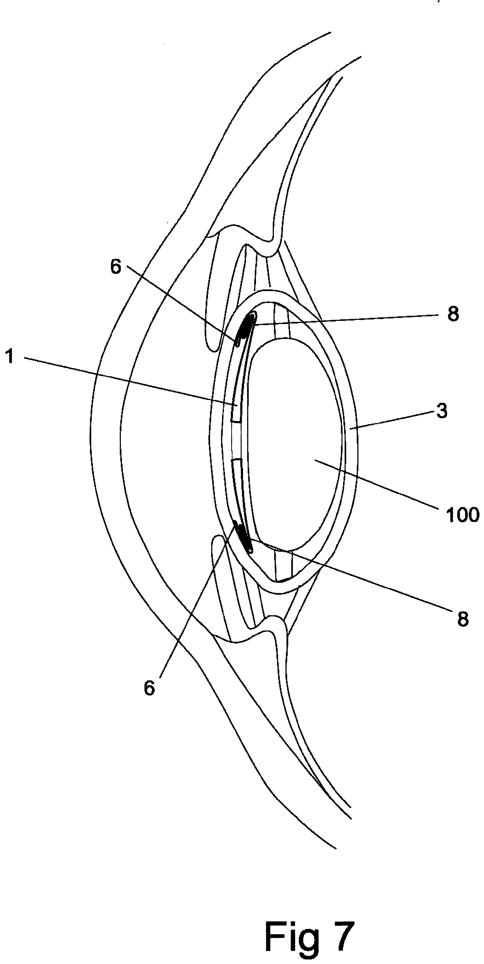

The supplementary intraocular lens (1) of the invention comprises:
- a lenticular body (2) of a diameter similar to the inner diameter of the capsular bag (3) of the lens (see figure 7) or of the sulcus (4) (see figure 6),
- whose thickness is between 0.05-0.5 millimetres, and
- comprising also an extender element of said lenticular body (2), which functions as a stabilizer or securing element of the extended position of the body (2).

Very preferably, the lenticular body (2) has a diameter between 9 and 13 millimetres, to adapt correspondingly to the size of the capsular bag (3) (which is normally around 10 millimetres) and to the size of the sulcus (which is normally around 12 millimetres), while the preferred thickness of the lenticular body (2) would be between 0.05 and 0.35 millimetres, which is where the best compromise has been found between rigidity-flexibility, stability with the extender element, and ease of introduction, being very small.

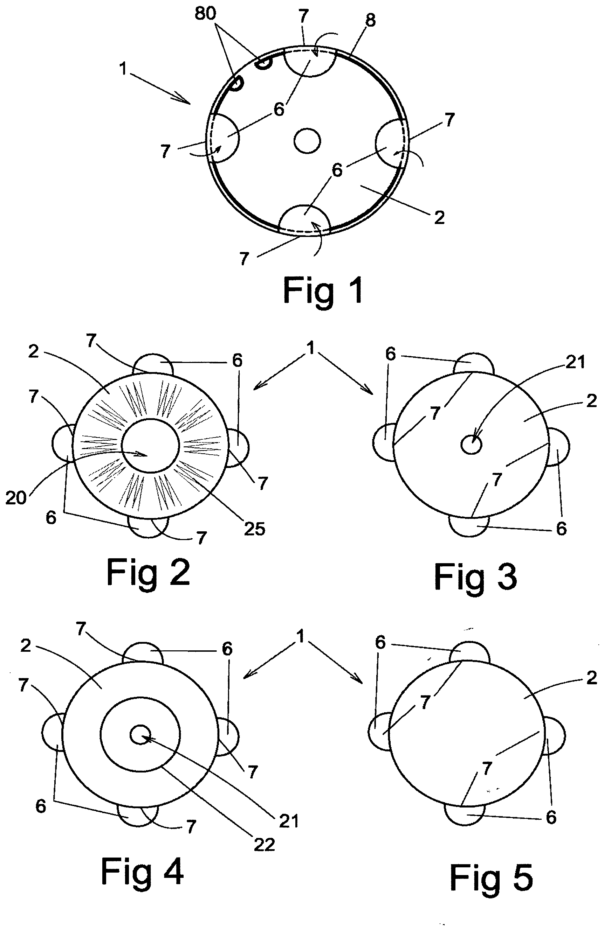

For its part, the most preferred embodiment of the extender element comprises a plurality of perimeter haptic handles (6) (see figures 1 to 5) (in any number) emerging from the edge of the lenticular body (2) through flexible joints (7) allowing them to bend backward, and a conventional flexible and elastic capsular ring (8) of similar diameter to that of the lenticular body (2) and which secures the haptic handles (6) when they are bent (see figs 1, 6 and 7). In this way the placement of the ring in the haptic handles (6) previously bent towards the inside of the body (2), and the extension of the ring (8) keeps the body (2) unfolded, its stability being ensured by the support of the assembly against the edges of the sulcus (4) or capsular bag (3). The ring (8) is preferably open, since it will thus adapt to different sizes of sulcus and capsular bags. It may comprise one or more eyelets (80) for handling. It is a capsular ring known to all ophthalmologists.

In a first variant of the invention provided mainly for the correction of deficiencies in the iris (see figure 2), the lenticular body (2) comprises a central pupil orifice (20), of a diameter similar to that of a pupil in mid-aperture. Ideally, the lenticular body (2) is coloured (25) like a natural iris with the exception of said central pupillary orifice (20).

In another variant of the invention that has been seen to correct astigmatism (see figure 3), the lenticular body (2) comprises a pinhole (21) (diameter about 1.36 mm for example, and therefore much smaller than the pupil orifice (20)). Indeed, in situations of incorrigible irregular astigmatism, we would use our supplementary lens and implement the pinhole, which may be the only way to correct an irregular astigmatism due to corneal scars.

In another variant of the invention that has been seen to correct presbyopia or ametropia, the lenticular body (2) has an opaque circular screen (22) (see figure 4) in the centre of which the pinhole (21) is arranged, the circular screen (22) having a diameter of approximately 3.25 millimetres, for example. In this variant the supplementary lens is not totally opaque as in versions in which it acts as an iris, but instead is totally transparent except for the small circular screen (22) with its pinhole (21). As in the previous versions it can go in the capsular sac (3) or sulcus (4) depending on whether the patient is phakic (in which case we would operate the lens in the same procedure) or pseudophakic. Unlike other intraocular lenses with a classic arrangement (centring haptic expansive handles) and graduated that carry this pinhole surrounded by an opaque circle, our supplementary lens (1) is not graduated, and overlaps another intraocular lens with graduation (100) (see figures 6 and 7), such that it is easier to explant in case of intolerance by the patient. Our supplementary (1) lens has the added advantage that it can be used in pseudophakic patients (already implanted with IOLs) while other lenses cannot, since a lens designed to go in the sac is too thick to be placed in sulcus without causing complications.

In all versions with pupillary orifice (20) or pinhole (21), the aqueous humour can also circulate through it (mainly in the version placed in the sulcus).

In' another variant of the supplementary lens of the invention, the lenticular body (2) is completely opaque (see figure 5) and lacks a central hole or orifice, usable in situations of incorrigible diplopia.

As indicated, the lenticular body (2) may lack graduation, which will be the normal situation, but not excluding implementation of some graduation if it is considered necessary, although it would not be necessary for the intended functions:
- IRIS function,
- Pinhole function,
- Pupil occluder function in incorrigible diplopia.

It should also be noted that the lenticular body (2) can be preferably made from a hydrophilic material such as hydrogel polymers, silicone etc., all of which are used in the manufacture of intraocular lenses.

Having sufficiently described the nature of the invention, as well as the manner in which it is carried out in practice, it should be noted that the provisions indicated above and shown in the accompanying drawings are subject to modifications in details insofar as they do not alter the essence of the invention.

## Claims

1. Supplementary intraocular lens (1) **characterized in that** it comprises:
- a lenticular body (2) of a diameter similar to the inner diameter of the capsular bag (3) of the lens or sulcus (4),
- whose thickness is between 0.05-0.5 millimetres, and
- and an extender element of said lenticular body (2).

2. Supplementary intraocular lens (1) according to claim 1, **characterised in that** the lenticular body (2) has a diameter between 9 and 13 millimetres.

3. Supplementary intraocular lens (1) according to any of the preceding claims, **characterised in that** the thickness of the lenticular body (2) is between 0.05 and 0.35 millimetres.

4. Supplementary intraocular lens (1) according to any of the preceding claims, **characterised in that** the extender element comprises a plurality of peripheral haptic handles (6) emerging from the edge of the lenticular body (2) through flexible joints (7) so that they can bend backward, and a flexible elastic capsular ring (8) of a similar diameter to the lenticular body (2) and which secures the haptic handles (6) when they are bent.

5. Supplementary intraocular lens (1) according to claim 4, **characterised in that** the ring (8) is open.

6. Supplementary intraocular lens (1) according to claim 5, **characterised in that** the open ring (8) comprises eyelets (80) for handling.

7. Supplementary intraocular lens (1) according to any of the preceding claims, **characterised in that** the lenticular body (2) comprises a central pupil orifice (20).

8. Supplementary intraocular lens (1) according to claim 7, **characterised in that** the lenticular body (2) is coloured like a natural iris with the exception of the central pupillary orifice (20).

9. Supplementary intraocular lens (1) according to any one of claims 1 to 6, **characterised in that** the (2) lenticular body comprises a pinhole (21).

10. Supplemental intraocular lens (1) according to claim 9, **characterised in that** the pinhole (21) has a diameter of 1.36 millimetres

11. Supplementary intraocular lens (1) according to any of claims 9 or 10, **characterised in that** the lenticular body (2) has an opaque circular screen (22) in the centre of which the pinhole (21) is arranged

12. Supplementary intraocular lens (1) according to claim 11, **characterised in that** the circular screen (22) has a diameter of approximately 3.25 millimetres

13. Supplementary intraocular lens (1) according to any one of claims 1 to 6, **characterised in that** the (2) lenticular body is completely opaque and lacks a central hole or orifice.

14. Supplementary intraocular lens (1) according to any of the preceding claims, **characterised in that** the (2) lenticular body has no graduation.

15. Supplementary intraocular lens (1) according to any of the preceding claims, **characterised in that** the lenticular body (2) is made of hydrophilic material.
